(19) Europäisches Patentamt
European Patent Office
Office européen des brevets

(11) Publication number: 0 061 128
B1

(12) EUROPEAN PATENT SPECIFICATION

(45) Date of publication of patent specification: 04.09.85

(21) Application number: 82102123.5

(22) Date of filing: 16.03.82

(51) Int. Cl.[4]: C 07 D 213/38, C 07 D 455/04, C 07 D 213/55, C 07 D 409/14, C 07 D 407/14, C 07 D 401/10, C 07 D 409/04, C 07 D 401/04, C 07 D 407/04, B 41 M 5/12

(54) **Pyridine compounds, process for their preparation and systems containing them.**

(30) Priority: 16.03.81 US 243798
16.03.81 US 244490

(43) Date of publication of application:
29.09.82 Bulletin 82/39

(45) Publication of the grant of the patent:
04.09.85 Bulletin 85/36

(84) Designated Contracting States:
CH DE FR GB LI

(56) References cited:
GB-A-2 029 591
US-A-3 985 376

(73) Proprietor: STERLING DRUG INC.
90 Park Avenue
New York New York (US)

(72) Inventor: Schmidt, Paul Joseph
3590 Concerto Drive
Sharonville Ohio (US)
Inventor: Hung, William Mo-Wei
9176 Millcliff Drive
Cincinnati Ohio (US)

(74) Representative: Baillie, Iain Cameron et al
c/o Ladas & Parry Isartorplatz 5
D-8000 München 2 (DE)

EP 0 061 128 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a group of compounds classified in the field of organic chemistry as 4-aryl or heteroaryl-2,6-bis(heteroaryl) or [(substituted-amino)phenyl]pyridines useful as color formers in pressure-sensitive carbonless duplicating systems and thermal marking systems, to a process for the preparation thereof and to pressure-sensitive carbonless duplicating systems and thermal marking systems containing the same.

Several classes of organic compounds of widely diverse structural types are known to be useful as color formers for carbonless duplicating systems. Among the more important classes are the phenothiazines, for example N-benzoyl leuco methylene blue; fluorans, for example 2'-anilino-6'-diethylaminofluroan; phthalides, for example crystal violet lactone; arylsulfinate salts of Michler's Hydrol; substituted phenylpyridines and various other types of colorless precursors currently emloyed in commercially accepted carbonless copy systems. Typical of the many such are those described in U.S. Patents 2,712,507, 2,800,457, 3,041,289, and 4,000,087. Many of the color formers in the prior art suffer one or more disadvantages such as low tinctorial strength, poor light stability, poor xerographic copiability and low solubility in common organic solvents, the latter disadvantage thus requiring the use of specialized and expensive solvents in order to obtain microencapsulated solutions of sufficient concentration for use in pressure-sensitive copy systems.

The following appear to constitute the most pertinent prior art relative to the present invention.

U.S. Patent 3,985,376 discloses compounds of the formula:

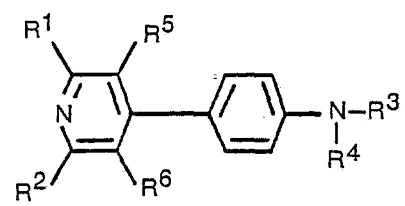

in which

$R^1$ and $R^2$ are hydrogen or alkyl or aryl of one to eight carbon atoms which may bear alkoxy or halogen as a substituent;

$R^3$ is hydrogen or alkyl of one to five carbon atoms;

$R^4$ is alkyl, haloalkyl, cyanalkyl, aryl or aralkyl of one to eight carbon atoms which may bear alkoxy as a substituent;

$R^5$ and $R^6$ are hydrogen or carbalkoxy of two to five carbon atoms and $R^3$ And $R^4$ may be closed to form a ring. Specific compounds disclosed are those wherein $R^1$ and $R^2$ are each phenyl, $R^5$ and $R^6$ are each hydrogen, $R^3$ is methyl and $R^4$ is methyl, phenyl or *p*-ethoxyphenyl, and also those wherein $R^1$ and $R^2$ are each *p*-methoxyphenyl, $R^5$ and $R^6$ are each hydrogen and $R^3$ and $R^4$ are each methyl. The compounds are stated to be useful as dye precursors for pressure-sensitive recording material.

Frank and Seven, J. Amer. Chem. *71,* 2629—2635 (1949) discloses 4-(*p*-chlorophenyl)-2,6-diphenylpyridine, 4-anisyl-2,6-diphenylpyridine and 2,6-diphenyl-4-(*m*-methoyphenyl)pyridine. These compounds were isolated as reaction products in a study of the Chichibabin reaction.

M. Weiss, J. Amer. Chem. Soc. *74,* 200—202 (1952) discloses in most pertinent part compounds of the formula

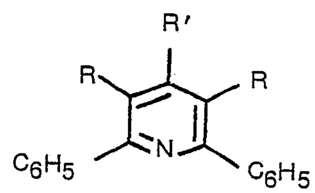

wherein R is hydrogen, and R' is phenyl, 4-methoxyphenyl, 3,4-methylenedioxyphenyl, 4-dimethylaminophenyl or 2-methoxyphenyl. These compounds were isolated as reaction products in a study of the Chichibabin synthesis.

E. Koenigs and E. Ruppelt, Ann. *509,* 142—158 (1934) disclose as basic dyestuffs a number of 4-[*p*-dialkylaminophenyl]-pyridines.

Gilman et al., J. Org. Chem. *22,* 1169—1171 (1957) in most pertinent part disclose 2,6-bis(*p*-diethylaminophenyl)pyridine as a relatively weak liquid scintillator solute.

United Kingdom Patent Application 2,029,591A discloses pyridine derivatives having the formula

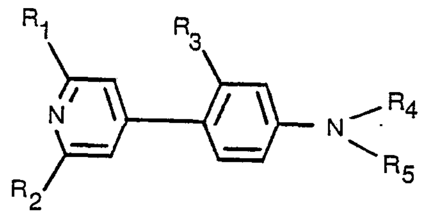

wherein

$R_1$ and $R_2$ are independently hydrogen, phenyl group or chlorine-substituted phenyl groups;

$R_3$ is hydrogen, lower-alkyl groups or lower-alkoxy groups;

$R_4$ and $R_5$ are independently lower-alkyl groups, benzyl group or phenyl group, said lower-alkyl groups possibly being substituted with a cyano group, a chlorine atom or a lower-alkoxy group. The compounds are stated to produce a yellow color on heating in the presence of an electron acceptor.

The present invention provides novel 4-aryl- or heteroaryl-2,6-bis(heteroaryl) or [(substituted-amino)phenyl]pyridines useful as color formers in pressure-sensitive carbonless duplicating systems and thermal marking systems. The compounds develope light-stable colored images of good tinctorial strength, and are soluble in common organic solvents. Moreover, because the compounds produce yellow to orange colors, they are especially valuable as toners used in admixture with other color formers to produce images of a neutral shade.

The present invention relates to a compound having Formula I hereinbelow:

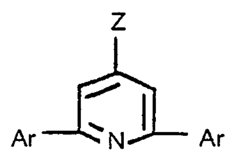

I

wherein:

Ar is a substituent having the formula

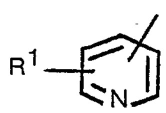 , 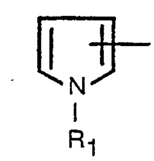 , 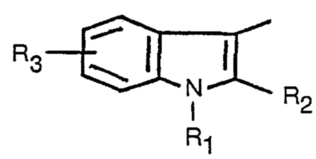

(a) (b) (c)

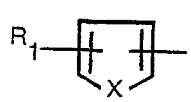 or 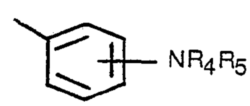

(d) (e)

in which:

$R_1$ is hydrogen or non-tertiary $(C_1—C_4)$-alkyl;

$R_2$ is hydrogen, phenyl or non-tertiary $(C_1—C_4)$-alkyl;

$R_3$ is hydrogen, non-tertiary $(C_1—C_4)$-alkyl or non-tertiary $(C_1—C_4)$-alkoxy;

$R_4$ and $R_5$ are the same or different and are $(C_1—C_4)$-alkyl or benzyl; or $NR_4R_5$ is pyrrolidinyl, piperidinyl, or morpholinyl;

X is O or S;

Z is 9-julolidinyl or a substituent having the formula

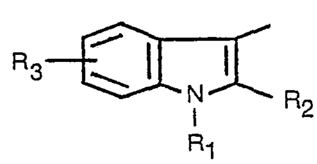

and when Ar is

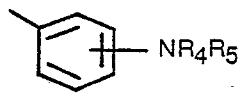

(f)

then Z in addition may be naphthyl or a substituent having the formula

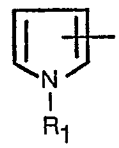

(b)

,

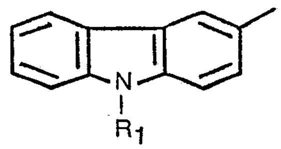

,

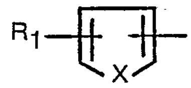

(d)

or

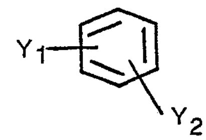

(g)

wherein:

$Y_1$ and $Y_2$ are the same or different and are selected from hydrogen, $(C_1$—$C_4)$-alkyl, $(C_1$—$C_4)$-alkoxy, halo, nitro, $(C_1$—$C_4)$-alkoxy-carbonyl; phenyl or $NR_9R_{10}$;

$R_9$ is $(C_1$—$C_4)$-alkyl or benzyl;

$R_{10}$ is $(C_1$—$C_4)$-alkyl, benzyl, cyano-$(C_1$—$C_4)$-alkyl or

$NR_9R_{10}$ is pyrrolidinyl, piperidinyl, morpholinyl or isoindolinyl;

and when Ar is other than

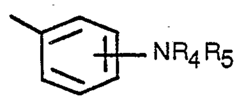

then Z in addition may be

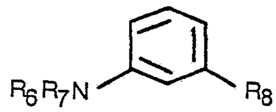

wherein:

$R_6$ is $(C_1$—$C_4)$-alkyl, and

$R_7$ is $(C_1$—$C_4)$-alkyl, benzyl, cyano-$(C_1$—$C_4)$-alkyl or

$NR_6R_7$ is pyrrolidinyl, piperidinyl, morpholinyl or isoindolinyl, and

$R_8$ is hydrogen, $(C_1$—$C_4)$-alkyl, halo, $(C_1$—$C_4)$-alkoxy, $(C_1$—$C_4)$-alkoxy-carbonyl or di-$(C_1$—$C_4)$-alkylamino.

A particular embodiment of the invention resides in a compound having Formula I hereinabove wherein Ar is of the formula (f) in which $R_4$ and $R_5$ have the previously given meanings and Z is a substituent having the formula

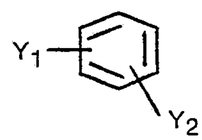

Preferred compounds within this embodiment are those wherein $Y_1$ and $Y_2$ are the same or different and are selected from hydrogen, $(C_1$—$C_4)$-alkyl and $(C_1$—$C_4)$-alkoxy and were $R_4$ and $R_5$ are each $(C_1$—$C_4)$-alkyl, especially 2,6-bis[4-(dimethylamino)phenyl]-4-phenylpyridine, 2,6-bis[4-(dimethylamino)phenyl]-4-(4-methylphenyl)pyridine and 2,6-bis[4-(dimethylamino)phenyl]-4-(4-methoxyphenyl)pyridine. Also preferred are the compounds wherein $R_4$ and $R_5$ are each $(C_1$—$C_4)$-alkyl and one of $Y_1$ and $Y_2$ is $NR_9R_{10}$, $R_9$ and $R_{10}$ each being $(C_1$—$C_4)$-alkyl, especially 2,4,6-tris[4-(dimethylamino)phenyl]pyridine. These compounds are particularly valuable because they are easily prepared from inexpensive and readily available starting materials.

A further particular embodiment, for the same reasons as the above, resides in a compound having Formula I hereinabove wherein Ar is a substituent having the formula

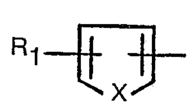

and Z is a substituent having the formula

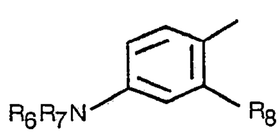

Preferred compounds of this embodiment are those wherein $R_1$ and X have the previously given meanings, $R_8$ is hydrogen or halo and $R_6$ and $R_7$ are each ($C_1$—$C_4$)-alkyl, especially 4-[4-(dimethylamino)phenyl]-2,6-bis(2-thienyl)pyridine, 4-[4-dimethylamino)phenyl]-2,6-bis(2-furyl)pyridine and 4-[2-chloro-4-(dimethylamino)phenyl]-2,6-bis(2-thienyl)-pyridine.

The present invention also resides in a process for preparing the compounds of Formula I hereinabove which comprises reacting approximately 2 molar equivalents of a ketone having Formula II hereinbelow:

$$Ar—COCH_3 \qquad \text{Formula II}$$

with approximately 1 molar equivalent of an aryl or heteroaryl aldehyde having Formula III hereinbelow:

$$Z—CHO \qquad \text{Formula III}$$

in the presence of ammonia or an ammonia-releasing agent where in Formulas II and III, Ar and Z have the previously given meanings.

The invention further resides in a pressure-sensitive carbonlesss duplicating system or thermal marking system containing a support sheet coated with a color-forming substance comprising a compound having Formula I hereinabove.

A particular embodiment resides in a pressure-sensitive transfer sheet adapted for use with a receiving sheet having an electron-accepting layer comprising a support sheet coated on one side with a layer of pressure-rupturable microcapsules, said microcapsules containing a liquid solution of a color-forming substance comprising at least one compound having Formula I.

Another particular embodiment resides in a heat-responsive record material comprising a support sheet coated on one side with a layer containing a mixture comprising at least one color-forming compound having Formula I and an acidic developer arranged such that application of heat will produce a mark-forming reaction between the color-forming compound and the acidic developer.

A further embodiment resides in a pressure-sensitive carbonless duplicating system or a thermal marking system containing a support sheet coated with a color-forming substance comprising a compound having Formula I hereinabove in combination with a blue or green and a red or orange color former.

Preferred systems within the particular embodiments above-described are those having a color-forming component of Formula I hereinabove in which $R_4$ and $R_5$ have the previously given meanings and Z is a substituent having the formula

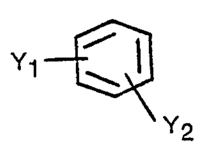

especially where $Y_1$ and $Y_2$ are the same or different and are hydrogen, ($C_1$—$C_4$)-alkyl or ($C_1$—$C_4$)-alkoxy, or where one of $Y_1$ and $Y_2$ is di-($C_1$—$C_4$)-alkylamino and where $R_4$ and $R_5$ are each ($C_1$—$C_4$)-alkyl.

Other preferred systems are those having a color-forming component of Formula I hereinabove in which Z is a substituent having the formula

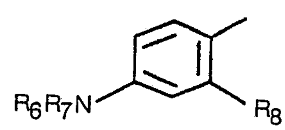

especially were $R_8$ is hydrogen or halo and $R_6$ and $R_7$ are each ($C_1$—$C_4$)-alkyl, and where Ar in Formula I is a substituent having the formula

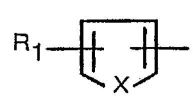

$R_1$ and X having the previously given meanings.

As used herein the term "halo" includes fluoro, chloro, bromo and iodo.

In the terms "($C_1$—$C_4$)-alkyl", "cyano-($C_1$—$C_4$)-alkyl", "($C_1$—$C_4$)-alkoxy" and "($C_1$—$C_4$)-alkoxy-carbonyl", "($C_1$—$C_4$)-" denotes a saturated acyclic alkyl moiety having from 1 to 4 carbon atoms such as methyl, ethyl, propyl, isopropyl, butyl, *sec*-butyl or isobutyl.

The terms "isoindolinyl" and "9-julolidinyl", of course, refer respectively to the radicals having the formulas

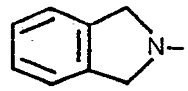

and

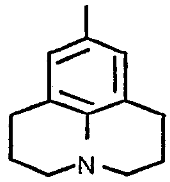

In accordance with the process of this invention the compounds having Formula I hereinabove are obtained by reacting approximately two molar equivalents of a ketone of Formula II hereinabove with approximately one molar equivalent of an aryl or heteroaryl aldehyde of Formula III hereinabove in the presence of ammonia or an ammonia-releasing agent. Thus, the ketone and aldehyde can be condensed in the presence of aqueous or alcoholic ammonia or alternatively, and preferably, in the presence of an ammonia-releasing agent such as ammonia acetate on acetic acid. The reactants are heated at about 50—150°C. for approximately 1 to 5 hours, usually at the reflux temperature of the solvent for about 2 hours. The product thus obtained can be isolated by filtration if it is insoluble in the reaction medium, or by dilution of the reaction medium with a miscible solvent in which the product is insoluble such as water or a lower-alkanol, for example, isopropyl, alcohol, or a mixture of these in order to effect precipitation of the product. Alternatively, the reaction mixture can be poured into water or dilute ammonium hydroxide and the product extracted with an organic solvent such as benzene toluene or chloroform followed by evaporation of the organic solvent leaving the product as a residue. Frequently the product begins to separate from the reaction mixture as an oil. Usually the addition of a lower-alkanol such as ethanol or isopropyl alcohol to the hot reaction will induce the product to separate in crystalline form on cooling. The product once isolated can be purified by conventional means such as trituration or recrystallization from a suitable solvent.

The ketones of Formula II and the aldehydes of Formula III which are required as starting materials in the preparation of the final product of Formula I are generally known and are either commercially available or readily obtained by conventional procedures well known in the art. Those ketones and aldehydes which are specifically novel can be prepared in accordance with the procedures described for preparation of the known compounds.

The novel compounds of Formula I hereinabove are pale yellow to colorless in the depicted form. When contracted with an acidic medium, for example silic gel, or one of the types ordinarily employed in pressure-sensitive carbonless duplicating systems such as silton clay or phenolic resins, the compounds of Formula I develop an intense yellow to orange image which is xerographically copiable and light stable. The compounds are thus highly suitable for use as color precursors, that is, color-forming substances in pressure-sensitive carbonless duplicating systems. Because they produce a yellow to orange color, these compounds are especially valuable as toners which are used in admixture with other color formers to produce images of a neutral shade which desirably possess an excellent xerographic copiability. Thus, a yellow to orange color former of Formula I hereinabove can be combined with a red or orange color former and a blue or green color former to afford a color-forming substance which produces a neutral or black image.

The compounds of this invention may be incorporated in any of the commercially accepted systems known in the carbonless duplicating art. A typical technique for such application is as follows: solutions containing one or more color precursor compounds of Formula I, optionally in admixture with other color formers as noted above, in suitable solvents are microencapsulated by well-known procedures, for example as described in U.S. Patents 3,649,649, 3,429,827 and 4,000,087. The microcapsules are coated on the reverse side of a transfer sheet with the aid of a suitable binder and the coated transfer sheet is then assembled in a manifold with the microcapsule-coated side in contact with a receiving sheet coated with an electron accepting substance for example silicon clay or phenolic resin. Application of pressure to the manifold such as that exerted by a stylus, typewriter or other form of writing or printing causes the capsules on the reverse side to rupture. The solution of the color former released from the ruptured microcapsules flows to the receiving sheet and on contact with the acidic medium thereon forms a colored image. It is, of course, obvious that variants of this mode of application can be utilized. For example, the receiving sheet in a manifold can alternatively be coated with the subject compounds and the acidic developing agent can be contained in microcapsules applied on the reverse side of the top sheet in the manifold; or the receiving sheet can be coated with a mixture containing both the acidic developing agent and the microencapsulated color former.

It has also been found that when the compounds of Formula I are intimately mixed with an acidic developer of the type generally employed in thermal papers such as described in U.S. Patents 3,539,375 and 3,447,944, that is, papers which produce a colored image when contacted with a heated stylus or heated type, for example, bisphenol A, heating of the mixture produces a colored image of varying shades from yellow to orange depending on the particular compound of the invention employed. As noted above, darker shades can be produced by mixing the compounds of Formula I with other color formers. The ability

of the compounds of Formula I to form an intense color when heated in admixture with an acidic developer such as bisphenol A makes them useful in thermal paper marking systems either where an original or duplicate copy is prepared by contacting the thermal paper with a heated stylus or heated type in any of the methods generally known in the art.

The molecular structure of the compounds of this invention were assigned on the basis of the modes of synthesis, elemental analysis and study of their infrared and nuclear magnetic responses spectra.

Example 1

A mixture containing 4.2 g. of 4-anisaldehyde, 9.8 g of 4-(dimethylamino)acetophenone, 36 g. of ammonium acetate and 50 ml. of glacial acetic acid was refluxed for one hour and was then allowed to stand at room temperature overnight. The mixture was filtered and the collected solid was washed with isopropanol and water. The solid was then slurried in 100 ml. of hot isopropanol. After cooling to room temperature, the pale yellow solid was collected, washed with isopropanol and dried to give 3.9 g. of 4-(4-methoxyphenyl)-2,6-bis[4-(dimethylamino)phenyl]pyridine, m.p. 194—197°C. This product produced an orange image on contact with acidic clay or phenolic resin.

Example 2

A mixture containing 3.2 g. of benzaldehyde, 9.8 g. of 4-(dimethylamino)acetophenone, 30 g. of ammonium acetate and 75 ml. of glacial acetic acid was refluxed for two hours. After cooling to room temperature, the reaction mixture was poured into 300 ml. of toluene and 400 ml. of 5% aqueous ammonium hydroxide. The toluene layer was separated, washed successively with water and saturated aqueous sodium chloride and evaporated to dryness under vacuum. The residue was slurried in ethanol and collected by filtration to give 2.1 g. of 4-phenyl-2,6-bis[4-(dimethylamino)-phenyl]pyridine, as a pale yellow solid, m.p. 176—179°C. A toluene solution of the product developed an orange image when contacted with an acidic clay and a yellow image when contacted with phenolic resin.

Example 3

Following a procedure similar to that described in Example 1 but employing 4.0 g. of 4-methylbenzylaldehyde, 9.8 g. of 4-(dimethylamino)acetophenone, 35 g. of ammonium acetate and 40 ml. of glacial acetic acid there was obtained 3.8 g. of 4-(4-methylphenyl-2,6-bis[4-(dimethylamino)-phenyl]pyridine, as a pale greenish yellow solid, m.p. 210—222°C. This product produced an orange image on contact with acidic clay or phenolic resin.

Example 4

Following a procedure similar to that described in Example 2 but employing 4.5 g. of 4-(dimethylamino)benzaldehyde, 9.8 g. of 4-(dimethylamino)acetophenone, 30 g. of ammonium acetate and 75 ml. of glacial acetic acid there was obtained 2.3 g. of 2,4,6-tris[4-(dimethylamino)phenyl]pyridine, as a yellow solid, m.p. 256—260°C. This product produced a yellow image on contact with acidic clay or phenolic resin.

Example 5

Following a procedure similar to that described in Example 2 but employing 4.2 g. of 4-chloronbenzaldehyde, 9.8 g. of 4-(dimethylamino)acetophenone, 36 g. of ammonium acetate and 50 ml. of glacial acetic acid there was obtained 2.4 g. of 4-(4-chlorophenyl)-2,6-bis[4-(dimethylamino)-phenyl]pyridine, as a pale yellow solid, m.p. 178—182°C. This product produced a yellowish-orange image on contact with acidic clay or phenolic resin.

Example 6

Following a procedure similar to that described in Example 2 but employing 5.5 g. of 2-chloro-4-(dimethylamino)benzaldehyde, 9.8 g. of 4-(dimethylamino)acetophenone, 35 g. of ammonium acetate and 50 ml. of glacial acetic acid there was obtained 3.4 g. of 4-[2-chloro-4-(dimethylamino)phenyl]-2,6-bis[4-(dimethylamino)-phenyl]pyridine, as a pale yellow solid, m.p. 131—139°C. The product produced a yellow image on contact with acidic clay or phenolic resin.

Example 7

Following a procedure similar to that described in Example 1 but employing 4.5 g. of 4-nitrobenzaldehyde, 9.8 g. of 4-(dimethylamino)acetophenone, 35 g. of ammonium acetate and 50 ml. of glacial acetic acid there was obtained 5.5 g. of 4-(4-nitrophenyl)-2,6-bis[4-(dimethylamino)-phenyl]pyridine, as a yellow solid, m.p. 156—164°C. This product produced an orange image on contact with acidic clay or phenolic resin.

Example 8

Following a procedure similar to that described in Example 2 but employing 5.6 g of 4-[(2-cyanoethyl) methylamino]-benzaldehyde, 9.8 g of 4-(dimethylamino) acetophenone, 40 g. of ammonium acetate and 35 ml. of glacial acetic acid there was obtained 3.5 g. of 4-[4-[(2-cyanoethyl) methylamino]phenyl]-2,6-bis[4-(dimethylamino)phenyl]pyridine as a light yellow solid, m.p. 170—174°C. This product produced an organge-yellow image on contact with acidic clay or phenolic resin.

Example 9

Following a procedure similar to that described in Example 2 but employing 5.0 g. of 2,5-dimethoxybenzaldehyde, 9.8 g. of 4-(dimethylamino) acetophenone, 40 g. of ammonium acetate and 30 ml. of glacial acetic acid, there was obtained 2.5 g. of 4-(2,5-dimethoxyphenyl)-2,6-bis[4-(dimethylamino)phenyl] pyridine, as a light yellow solid, m.p. 199—206°C. This product produced a yellowish orange image on contact with acidic clay or phenolic resin.

Example 10

A mixture containing 4.5 g. of 3-nitrobenzaldehyde, 9.8 g. of 4-(dimethylamino)acetophenone, 40 g. of ammonium acetate and 40 ml. of glacial acetic acid was refluxed for two hours. After cooling to room temperature, the resulting tarry solid was collected and was first slurried in isopropanol then in toluene to give 3.0 g. of 4-(3-nitrophenyl)-2,6-bis[4-(dimethylamino)-phenyl]pyridine, as a yellow solid, m.p. 194—200°C. This product produced a yellowish-orange image on contact with acidic clay or phenolic resin.

Example 11

A mixture containing 4.6 g of methyl 4-formylbenzoate, 9.8 g. of 4-(dimethylamino)acetophenone, 40 g. of ammonium acetate and 30 ml. of glacial acetic acid was heated 3 hours under reflux. The mixture was cooled to 70°C. and 75 ml. of isopropanol was added, reheated to 90—95°C. and held for 30 minutes. After cooling to room temperature, the yellow solid was collected, washed with isopropanol and dried to give 3.9 g. of 4-[4-(methoxycarbonyl)phenyl]-2,6-bis[4-(dimethylamino)phenyl]pyridine, m.p. 244—250°C. This product produced an orange image on contact with acidic clay or phenolic resin.

Example 12

Following a procedure similar to that described in Example 2 but employing 5.4 g. of 4-(diethylamino) benzaldehyde, 9.8 g. of 4-(dimethylamino)acetophenone, 30 g. of ammonium acetate and 75 ml. of glacial acetic acid there was obtained 1.0 g. of 4-[4-(diethylamino)phenyl]-2,6-bis[4-(dimethylamino)phenyl]-pyridine as a yellow solid, m.p. 193.9—201.8°C. A toluene solution of the product developed an orange image when contacted with acidic clay and a yellow image when contacted with phenolic resin.

Example 13

Following a procedure similar to that described in Example 1 but employing 4.0 g. of 4-biphenylcarboxaldehyde, 6.5 g. of 4-(dimethylamino)acetophenone, 27 g. of ammonium acetate and 25 ml. of glacial acetic acid there was obtained 3.0 g. of 4-(4-biphenylyl)-2,6-bis[4-(dimethylamino)phenyl] pyridine, as a yellow solid, m.p. 213—216°C. This product produced an orange image on contact with acidic clay or phenolic resin.

Example 14

Following a procedure similar to that described in Example 1 but employing 4.7 g. of 1-naphthaldehyde, 9.8 g. of 4-(dimethylamino)acetophenone, 40 g. of ammonium acetate and 35 ml. of glacial acetic acid there was obtained 3.6 g. of 4-(1-naphthyl)-2,6-bis[4-(dimethylamino)phenyl]pyridine, as a light yellow solid, m.p. 189—208°C. A chloroform solution of the product developed an orange image when contacted with acidic clay and a yellow image when contacted with phenolic resin.

Example 15

Following a procedure similar to that described in Example 2 but employing 6.0 g. of 4-(1-pyrrolidinyl) benzaldehyde, 11 g. of 4-(dimethylamino)acetophenone, 40 g. of ammonium acetate and 50 ml. of glacial acetic acid there was obtained 4.7 g of 4-[4-(1-pyrrolidinyl)phenyl]-2,6-bis [4-((dimethylamino)phenyl]-pyridine, as a yellow solid, m.p. 199.8—214.9°C. This product produced a yellow image on contact with acidic clay or phenolic resin.

Example 16

Following a procedure similar to that described in Example 1 but employing 6.3 g. of 4-(1-piperidinyl) benzaldehyde, 11 g. of 4-(dimethylamino)acetophenone, 40 g. of ammonium acetate and 50 ml. of glacial acetic acid there was obtained 5.2 g. of 4-[4-(1-piperidinyl)phenyl-2,6-bis[4-(dimethylamino)phenyl]-pyridine as a yellow solid, m.p. 217.5—220.9°C. A chloroform solution of the product developed an orange image when contacted with acidic clay and a yellow image when contacted with phenolic resin.

Example 17

Following a procedure similar to that described in Example 1 but employing 5.6 g. of 4-(2-isoindolinyl) benzaldehyde, 8.5 g. of 4-(dimethylamino)acetophenone, 40 g. of ammonium acetate and 50 ml. of glacial acetic acid there was obtained 4.0 g. of 4-[4-(2-isoindolinyl)phenyl]-2,6-bis [4-(dimethylamino)phenyl]-pyridine, as a muddy yellow solid, m.p. 265—271°C. A chloroform solution of the product developed an orange image when contacted with acidic clay and a yellow image when contacted with phenolic resin.

Example 18

Following a procedure similar to that described in Example 11 but employing 3.0 g. of 4-(4-morpholinyl) benzaldehyde, 5.0 g. of 4-(dimethylamino)acetophenone, 25 g. of ammonium acetate and 40 ml. of glacial acetic acid there was obtained 2.2 g. of 4-[4-(4-morpholinyl)phenyl]-2,6-bis[4-(dimethylamino)phenyl]pyridine as a pale yellow solid, m.p. 231—234°C. A chloroform solution of the product developed an orange image when contacted with acidic clay and a yellow image when contacted with phenolic resin.

Example 19

Following a procedure similar to that described in Example 2 but employing 4.8 g. of 2-methyl-4-(1-pyrrolidinyl)-benzaldehyde, 8.5 g. of 4-(dimethylamino) acetophenone, 40 g. of ammonium acetate and 50 ml. of glacial acetic acid there was obtained 2.6 g. of 4-[2-methyl-4-(1-pyrrolidinyl)phenyl]-2,6-bis-[4-(dimethylamino)phenyl] pyridine, as a yellow solid, m.p. 225—229°C. A chloroform solution of the product developed an orange image when contacted with acidic clay and a yellow image when contacted with phenolic resin.

Example 20

Following a procedure similar to that described in Example 2 but employing 5.1 g. of 9-julolidinecarboxaldehyde, 8.2 g. of 4-(dimethylamino)acetophenone, 33 g. of ammonium acetate and 25 ml. of glacial acetic acid there was obtained 3.5 g. of 4-(9-julolidinyl)-2,6-bis[4-(dimethylamino)phenyl pyridine, as an orange-yellow solid, m.p. 237—249°C. This product produce a yellowish-orange image on contact with acidic clay or phenolic resin.

Example 21

Following a procedure similar to that described in Example 11 but employing 5.1 g. of 4-anisaldehyde, 9.8 g. of 3-(dimethylamino)acetophenone, 40 g. of ammonium acetate and 35 ml. of glacial acetic acid there was obtained 2.5 g. of 4-(4-methoxyphenyl)-2,6-bis[3-(dimethylamino)phenyl] pyridine, as a milky white solid, m.p. 187—189°C. This product produced a weak yellow image on contact with acidic clay or phenolic resin.

Example 22

Following a procedure similar to that described in Example 2 but employing 3.2 g. of benzaldehyde, 9.8 g. of 3-(dimethylamino)acetophenone, 40 g. of ammonium acetate and 35 ml. of glacial acetic acid there was obtained 2.0 g. of 4-phenyl-2,6-bis[3-(dimethylamino)phenyl]pyridine, as an off-white solid, m.p. 142—147°C. This product produced a weak orange image on contact with acidic clay or phenolic resin.

Example 23

Following a procedure similar to that described in Example 2 but employing 3.6 g. of 4-methylbenzaldehyde, 11.5 g of 4-(diethylamino)acetophenone, 40 g. of ammonium acetate and 30 ml. of glacial acetic acid there was obtained 2.0 g. of 4-(4-methylphenyl)-2,6-bis[4-(diethylamino)phenyl] pyridine, as a yellow solid, m.p. 174—178.5°C. This product produced an orange image on contact with acidic clay or phenolic resin.

Example 24

Following a procedure similar to that described in Example 2 but employing 4.1 g. of 4-anisaldehyde, 11.5 g. of 4-(diethylamino)acetophenone, 40 g. of ammonium acetate and 30 ml. of glacial acetic acid there was obtained 2.0 g. of 4-(4-methoxyphenyl)-2,6-bis[4-(diethylamino)phenyl]pyridine as a brownish-yellow solid, m.p. 77—87.5°C. This product produced a yellowish-orange image on contact with acidic clay or phenolic resin.

Example 25

Following a procedure similar to that described in Example 11 but employing 4.1 g. of 4-anisaldehyde, 21 g. of 4-(dibenzylamino)acetophonone, 40 g. of ammonium acetate and 30 ml. of glacial acetic acid there was obtained 5.5 g. of 4-(4-methoxyphenyl)-2,6-bis[4-(dibenzylamino)phenyl] pyridine as a yellow solid, m.p. 72.3—77.5°C. This product produced a yellow image on contact with acidic clay or phenolic resin.

Example 26

Following a procedure similar to that described in Example 2 but employing 3.4 g. of 4-anisaldehyde, 10.2 g. of 4-(1-piperidinyl)acetopenone, 35 g. of ammonium acetate and 40 ml. of glacial acetic acid there was obtained 3.4 g. of 4-(4-methoxyphenyl)-2,6-bis[4-(1-piperidinyl)phenyl] pyridine, as an off-white solid, m.p. 114—118°C. This product produced a yellow image on contact with acidic clay or phenolic resin.

Example 27

A mixture containing 4.6 g. of 4-anisaldehyde, 14 g. of 4-(4-morpholinyl)acetophenone, 50 g. of ammonium acetate and 60 ml. of glacial acetic acid was refluxed for two hours. After cooling to room

temperature, the solution was decanted and the residual tarry solid was washed with water and isopropanol. The tarry solid was then dissolved in 250 ml. of acetone and added dropwise to 1,500 ml. of water and 25 g. of sodium chloride to yield 8.5 g. of 4-(4-methoxyphenyl)-2,6-bis[4-(4-morpholinyl)-phenyl]-pyridine as a pale yellow solid, m.p. 100—110°C. This product produced a greenish-yellow image on contact with acidic clay or phenolic resin.

Example 28

Following a procedure similar to that described in Example 1 but employing 4.1 g. of 4-fluorobenzaldehyde, 12.6 g. of 4-(1-pyrrolidinyl)acetophenone, 50 g. of ammonium acetate and 60 ml. of glacial acetic acid there was obtained 2.9 g. of 4-(4-fluorophenyl)-2,6-bis[4-(1-pyrrolidinyl) phenyl]pyridine as a light yellow solid, m.p. 207—212°C. A chloroform solution of the product developed a reddish orange image when contacted with acidic clay and a orange image when contacted with phenolic resin.

Example 29

Following a procedure similar to that described in Example 2 but employing 3.6 g. of 2-thiophenecarboxaldehyde, 9.8 g. of 4-(dimethylamino)acetophenone, 35 g. of ammonium acetate and 40 ml. of glacial acetic acid there was obtained 3.6 g. of 4-(2-thienyl)-2,6-bis[4-((dimethylamino)phenyl] pyridine, as a straw-colored solid, m.p. 191—198°C. This product produced an orange image on contact with acidic clay or phenolic resin.

Example 30

Following a procedure similar to that described in Example 11 but employing 3.3 g. of N-methylpyrrole-2-carboxaldehyde, 9.8 g. of 4-(dimethylamino)acetophenone, 40 g. of ammonium acetate and 30 ml. of glacial acetic acid there was obtained 1.5 g. of 4-(1-methylpyrrol-2-yl)-2,6-bis[4-((dimethylamino)-phenyl]pyridine, as a greenish-yellow solid, m.p. 193—200°C. This product produced a yellow image on contact with acidic clay or phenolic resin.

Example 31

Following a procedure similar to that described in Example 11 but employing 3.3 g. of 5-methyl-2-furaldehyde, 9.8 g. of 4-(dimethylamino)acetophenone, 40 g. of ammonium acetate and 30 ml. of glacial acetic acid there was obtained 2.2 g. of 4(5-methyl-2-furyl)-2,6-bis[4-(dimethylamino) phenyl]pyridine, as a yellow solid, m.p. 219.3—225.5°C. This product produced an orange image on contact with acidic clay or phenolic resin.

Example 32

Following a procedure similar to that described in Example 11 but employing 4.4 g. of indole-3-carboxaldehyde, 9.8 g. of 4-(dimethylamino)acetophenone, 40 g. of ammonium acetate and 30 ml. of glacial acetic acid there was obtained 4 g. of 4-(indol-3-yl)-2,6-bis[4-dimethylamino)phenyl] pyridine, as a yellow solid, m.p. 237.2—242.0°C. This product produced a yellowish-orange image on contact with acidic clay or phenolic resin.

Example 33

Following a procedure similar to that described in Example 1 but employing 2.3 g. of N-ethyl-3-carbazolecarboxaldehyde, 3.3 g. of 4-(dimethylamino) acetophenone, 13.2 g. of ammonium acetate and 15 ml. of glacial acetic acid there was obtained 2.0 g. of 4-(9-ethylcarbazol-3-yl)-2,6-bis[4-((dimethylamino)-phenyl]pyridine as a yellow solid, m.p. 157—173°C. This product produced an orange image on contact with acidic clay or phenolic resin.

Example 34

Following a procedure similar to that described in Example 2 but employing 3.6 g. of indole-3-carboxaldehyde, 8.1 g. of 2-(dimethylamino)acetophenone, 34 g. of ammonium acetate and 30 ml. of glacial acetic acid there was obtained 3.1 g. of 4-(indole-3-yl)-2,6-bis[2-(dimethylamino)phenyl] pyridine, as a pale yellow solid, m.p. 230—231°C. This product produced a yellow image on contact with acidic clay or phenolic resin.

Example 35

Following a procedure similar to that described in Example 11 but employing 4.3 g. of indole-3-carboxaldehyde, 21 g. of 4-(dibenzylamino)acetophenone, 40 g. of ammonium acetate and 35 ml. of glacial acetic acid there was obtained 5.4 g. of 4-(indole-3-yl)-2,6-bis[4-(dibenzylamino)phenyl] pyridine, as a brownish-yellow solid, m.p. 105.0—111.8°C. This product produced a yellow image on contact with acidic clay or phenolic resin.

Example 36

Following a procedure similar to that described in Example 2 but employing 3.6 g. of indole-3-carboxaldehyde, 9.5 g. of 4-(1-pyrrolidinyl)acetophenone, 36 g. of ammonium acetate and 40 ml. of glacial

acetic acid there was obtained 4.1 g. of 4-(indol-3-yl)-2,6-bis[4-(1-pyrrolidinyl)phenyl] pyridine, as a tan solid, m.p. 145—147°C. This product produced a yellow image on contact with acidic clay or phenolic resin.

Example 37

Following a procedure similar to that described in Example 1 but employing 3.8 g. of 2-thiophenecarboxaldehyde, 13.5 g. of 4-(1-piperidinyl)acetophenone, 50 g. of ammonium acetate and 60 ml. of glacial acetic acid there was obtained 4.3 g. of 4-(2-thienyl)-2,6-bis[4-(1-piperidinyl)phenyl] pyridine, as a light yellow solid, m.p. 198—201°C. This product produced an orange color image on contact with acidic clay or phenolic resin.

Example 38

Following a procedure similar to that described in Example 1 but employing 4.9 g. of indole-3-carboxaldehyde, 14 g. of 4-(4-morpholinyl)acetophenone, 50 g. of ammonium acetate and 60 ml. of glacial acetic acid there was obtained 7.7 g. of 4-(indol-3-yl)-2,6-bis[4-(4-morpholinyl)phenyl] pyridine, as a tan solid, m.p. 270—275°C. This product produced a greenish yellow image on contact with acidic clay or phenolic resin.

Example 39

Following a procedure similar to that described in Example 2 but employing 4.8 g. of 2,4-bis(dimethyl-amino)benzaldehyde, 6.8 g. of 4-(dimethylamino)acetophenone, 33 g. of ammonium acetate and 25 ml. of glacial acetic acid there was obtained 0.3 g. of 4-[2,4-bis(dimethylamino)phenyl]-2,6-bis£[4-((dimethyl-amino)phenyl]pyridine, as a yellow solid, m.p. 235—260°C. This product produced a yellowish orange image on contact with acidic clay or phenolic resin.

Example 40

Following a procedure similar to that described in Example 2 but employing 11.5 g. of 2-ethoxy-4-(diethylamino)-benzaldehyde, 16.3 g. of 4-(dimethylamino)acetophenone, 65 g. of ammonium acetate and 40 ml. of glacial acetic acid there was obtained 3.1 g. of 4-[2-ethoxy-4-(diethylamino)phenyl]-2,6-bis[4-(dimethylamino)phenyl]pyridine, as a yellow solid, m.p. 100—110°C. This product produced a yellowish orange image on contact with acidic clay or phenolic resin.

Example 41

Following a procedure similar to that described in Example 2 but employing 5.0 g. of 4-fluorobenzaldehyde, 13.0 g. of 4-(dimethylamino)acetophenone, 53 g. of ammonium acetate and 40 ml. of glacial acetic acid there was obtained 5.1 g. of 4-(4-fluorophenyl)-2,6-bis[4-(dimethylamino)phenyl] pyridine, as a greenish-yellow solid, m.p. 185—187°C. This product produced a yellowish-orange image on contact with acidic clay or phenolic resin.

Example 42

Following a procedure similar to that described in Example 11 but employing 5.5 g. of the ethyl ester of 5-(dimethylamino)-2-formyl-benzoic acid, 8.1 g. of 4-(dimethylamino)-acetophenone, 30 g. of ammonium acetate and 25 ml. of glacial acetic acid there was obtained 2.7 g. of 4-[2-(ethoxycarbonyl)-4-(dimethyl-amino)phenyl]-2,6-bis[4-(dimethylamino)phenyl]pyridine as a yellow solid, m.p. 242.5—252.5°C. This product produced a yellow image on contact with acidic clay or phenolic resin.

Example 43

Following a procedure similar to that described in Example 2 but employing 4.4 g. of 1-ethyl-2-methylindole-3-carboxyaldehyde, 7.7 g. of 4-(dimethylamino)acetophenone, 30 g. of ammonium acetate and 25 ml. of glacial acetic acid there was obtained 2.8 g. of 4-(1-ethyl-2-methylindol-3-yl)-2,6-bis[4-(dimethylamino)phenyl]pyridine, as a light yellow solid, m.p. 216—224°C. This product produced a yellow image on contact with acidic clay or phenolic resin.

Example 44

A mixture containing 7.2 g of 4-(benzylethylamino)-benzaldehyde, 10.9 g. of 4-(dimethylamino)-acetophenone, 40 g. of ammonium acetate and 25 ml. of glacial acetic acid was heated three hours at 125°C. After cooling to room temperature, the reaction mixture was diluted with water and extracted with toluene. The toluene extracts were washed with water followed by saturated aqueous sodium chloride and evaporated to dryness under vacuum. The residual oil was crystallized by repeated trituration first with hexane and then with isopropyl alcohol. The resulting solid was collected by filtration, washed with isopropyl alcohol and dried to give 4.2 g of 4-[4-(benzylethylamino)phenyl]-2,6-bis[4-(dimethylamino)-phenyl]pyridine, m.p. 157—163.5°C. This product produced a yellow image on contact with acidic clay or phenolic resin.

It is contemplated that by following procedures similar to those described in Examples 1, 2, 10, 11, 27 and 44 but employing the appropriately substituted acetophenones and aryl or heteroaryl aldehydes of Formulas II and III, respectively, there will be obtained the 4-aryl- or heteroaryl-2,6-bis[(substituted-amino)phenyl] pyridines of Formula I, Examples 45—57 presented in Table A below:

TABLE A

| Ex. | Z | $R_1$ | $R_2$ | Position of $NR_1R_2$ |
|---|---|---|---|---|
| 45 | $C_6H_5$ | $(CH_3)_2CH$ | $(CH_3)_2CH$ | 4 |
| 46 | $C_6H_5$ | $CH_3$ | $C_2H_5$ | 4 |
| 47 | $C_6H_5$ | $CH_3$ | $C_4H_9$ | 4 |
| 48 | 5-$CH_3$-2-thienyl | $CH_3$ | $CH_3$ | 4 |
| 49 | 4-$(CH_3)_2CH$-$C_6H_4$ | $CH_3$ | $CH_3$ | 4 |
| 50 | 2-$C_2H_5O$-3-$CH_3O$-$C_6H_3$ | $CH_3$ | $CH_3$ | 4 |
| 51 | 3-Br-$C_6H_4$ | $CH_3$ | $CH_3$ | 2 |
| 52 | 4-Cl-3-$NO_2$-$C_6H_3$ | $C_2H_5$ | $C_2C_5$ | 4 |
| 53 | 4-*t*-$C_4H_9$-$C_6H_4$ | $CH_3$ | $CH_3$ | 4 |
| 54 | 4-$C_4H_9O$-$C_6H_4$ | $CH_3$ | $CH_3$ | 4 |
| 55 | 4-$C_4H_9O_2C$-$C_6H_4$ | $CH_3$ | $CH_3$ | 4 |
| 56 | 4-$(C_6H_5CH_2)_2N$-$C_6H_4$ | $CH_3$ | $CH_3$ | 2 |
| 57 | 4-$(C_4H_9)_2N$-$C_6H_4$ | $CH_3$ | $CH_3$ | 4 |

Example 58

A solution containing 0.73 g. of the color former of Example 3 in 30 g. of isopropylbiphenyl and a solution containing .5 g. of carboxymethylcellulose in 100 ml. of water were mixed and emulsified by rapid stirring. The desired particle size (5 microns) was checked by microscope. To the emulsion was added a solution containing 7.5 g. of pigskin gelatin in 60 ml. of water. The pH was adjusted to 6.5 with 10% aqueous sodium hydroxide with rapid stirring, and following the gradual addition of 335 ml. of water at 50°C., the pH was adjusted to 4.5 with 10% aqueous acetic acid with continued rapid stirring. After 5 minutes the mixture was cooled to 15°C., treated with 10 g. of 25% aqueous glutaraldehyde and rapidly stirred for 15 minutes. The resulting microcapsule dispersion was stirred more slowly overnight, diluted with water to 560 g. and coated on white typewriter paper sheets. The sheets were air-dried. Duplicate typwritten images were made on receiving sheets coated with either phenolic resin or acidic clay. The color former of Example 3 produced an orange image on both types of receiving sheets.

Example 59

To a solution prepared by dissolving 6.8 g. of terephthaloyl chloride in 37.1 g. of hot isopropylbiphenyl and then cooling to 30°C. was added a solution prepared by dissolving 2.1 g. of the color former of Example 1 in 34.3 g. of hot isopropylbiphenyl and then cooling to 40°C. The resulting mixture was slowly added to aqueous polyvinyl alcohol (prepared by diluting 5.9 g. of polyvinyl alcohol having a hydrolysis of 87 to 89% with 250 ml. of hot water, cooling to room temperature and further diluting with 40 ml. of water) and emulsified under high-shear agitation until a dispersed phase particle size of about 5 microns was obtained. The resulting emulsion was then stirred at conventional speed while adding a solution containing 2.2 g. of sodium carbonate and 3.9 g. of diethylenetriamine in 23 ml. of water. After stirring at room temperature for approximately 24 hours, the pH was adjusted to 7.3 with 15% aqueous sodium carbonate and the total weight of the suspension was adjusted to 326 g. by adding water if necessary. The resulting microcapsule suspension was then applied to a bond paper sheet (transfer sheet) and the coated sheet air-dried. The coated side of the transfer sheet was placed in contact with a receiving sheet coated with acidic clay. Writing on the transfer sheet produced an orange duplicate image on the receiving sheet.

Example 60

A polyvinyl alcohol dispersion of the color formers of Examples 2 and 31 were prepared by shaking 1 hour on a paint shaker a mixture contining 2.0 g. of the color former, 3.7 g. of water, 8.6 g. of 10% aqueous polyvinyl alcohol and 10 ml. of zirconium grinding beads. Meanwhile a polyvinyl alcohol dispersion of bisphenol A was prepared by shaking a mixture containing 9.8 g. of bisphenol A, 18.2 g. of water, 42 g. of 10% aqueous polyvinyl alcohol and 70 ml. of zirconium griding beads. The coating mixture was made by combining and thoroughly mixing 2.1 g. of the polyvinyl alcohol dispersion of the color former with 47.9 g.

of the polyvinyl alcohol dispersion of bisphenol A. The coating mixture was applied to white mimeo paper sheets and the sheets were dried at room temperature. Contacting the coated sheets with a heated stylus at a temperature between 100—150°C. produced a yellow image on the sheet coated with the color former of Example 2 and an orange image on the sheet coated with the color former of Example 31.

Example 61

A starch paste was prepared by stirring a mixture containing 15.2 g. of low-viscosity oxidized starch (sold under the tradename STAYCOM by A. E. Staley Manufacturing Co.) and 60.0 g. of distilled water at room temperature until the mixture became homogeneous. The mixture was heated with stirring at 90—93°C. for twenty minutes and then cooled to room temperature whereupon the resulting starch paste formed a gel. A starch dispersion of the color former of Example 1 was then prepared by shaking 0.5 hr. on a paint shaker a mixture containing 3.3 g. of the starch paste, 4.0 g. of the color former, 16 g. of distilled water, 17 g of glass shot and 4.0 g. of a solution prepared by combining 30 g. of anhydrous disodium phosphate and 5.6. g. of sodium hydroxide in 100 g. of distilled water. Meanwhile a benzoyl peroxide dispersion was prepared by homogenizing for 0.5 hr. a mixture containing 32.6 g. of the starch paste, 11.2 g. of low-viscosity oxidized starch, 5.2 g. of benzoyl peroxide, 21.6 g. of distilled water, 20 ml. of glass shot and 27 g of a solution prepared by combining 5.6 g. of sodium hydroxide and 30 g. of anhydrous disodium phosphate in 100 g. of distilled water. A paper coating mixture was prepared by combining and thoroughly mixing 14.5 g. of the starch dispersion of the color former with 26.2 g. of the starch dispersion of benzoyl peroxide. The resulting mixture was applied to white mimeo paper sheets and the sheets were dried at room temperature. Contacting the sheets with a heated stylus at a temperature between 100—150°C. produced an orange image.

Example 62

A mixture containing 7.5 g. of 4-(dimethylamino)benzaldehyde, 11 g. of 2-acetylfuran, 50 g. of ammonium acetate and 60 ml. of glacial acetic acid was refluxed for two hours. After cooling to room temperature, the reaction mixture was quenched with water and extracted with toluene. The toluene layer was separated, washed successively with water and saturated aqueous sodium chloride, and evaporated to dryness under vacuum. The residue was triturated from isopropanolhexane to give 2.3 g. of 4-[4-(dimethyl-amino)phenyl]-2,6-bis(2-furyl)pyridine, as a tan solid, m.p. 124—125°C. A toluene solution of the product developed an orange image when contacted with acidic clay and a yellow image when contacted with phenolic resin.

Example 63

A mixture containing 9.2 g. of 2-chloro-4-(dimethylamino)benzaldehyde, 13 g. of 2-acetylthiophene, 50 g. of ammonium acetate and 60 ml. of glacial acetic acid was heated under reflux 1.5 hours. The reaction mixture was cooled and the liquid was decanted. The tarry residue was broken up and slurried with 75 ml. of acetone. The solid was collected by filtration, reslurried in 100 ml. of acetone and filtered to give 2.9 g. of 4-[2-chloro-4-(dimethylamino)phenyl]-2,6-bis-(2-thienyl)pyridine, as a yellow solid, m.p. 147—150°C. This product produced a yellow image on contact with acidic clay or phenolic resin.

Example 64

Following a procedure similar to that described in Example 62 but employing 7.5 g of 4-(dimethylamino)benzaldehyde, 12.7 g. of 2-acetylthiophene, 50 g. of ammonium acetate and 60 ml. of glacial acetic acid there was obtained 2.8 g. of 4-[4-dimethylamino)phenyl]-2,6-bis(2-thienyl)pyridine as a yellow solid, m.p. 90—97°C. This product produced a yellow image in contact with acidic clay or phenolic resin.

Example 65

A mixture containing 6.0 g. of 4-(dimethylamino)benzaldehyde, 10 g. of 2-acetyl-1-methylpyrrole, 50 g. of ammonium acetate and 60 ml. of glacial acetic acid was heated 2 hours under reflux. After cooling to room temperature, 50 ml. of isopropanol was added and the resulting mixture was heated 1 hour under reflux. After cooling, the solid was collected by filtration, slurried in 100 ml. of isopropanol, and filtered to give 3.6 g. of 4-[4-(dimethylamino)phenyl]-2,6-bis(1-methylpyrrol-2-yl)pyridine, as a straw-yellow solid. This product produced a yellow image on contact with acidic clay or phenolic resin.

Example 66

Following a procedure similar to that described in Example 62 but employing 7.5 g. of 4-[(2-cyanoethyl)methylamino]benzaldehyde, 9.8 g. of 2-acetylthiophene, 53 g. of ammonium acetate and 40 ml. of glacial acetic there was obtained 4.6 g. of 4-[4-[(2-cyanoethyl)methylamino]phenyl]-2,6-bis-(2-thienyl)-pyridine as a brownish-yellow solid, m.p. 94—110°C. This product produced a yellow image on contact with acidic clay or phenolic resin.

Example 67

Following a procedure similar to that described in Example 62 but employing 7.5 g. of 4-(dimethylamino)benzaldehyde, 12.2 g. of 4-acetylpyridine, 50 g. of ammonium acetate and 60 ml. of glacial acetic acid there was obtained 6.4 g. of 4-[4-(dimethylamino)phenyl]-2,6-bis(4-pyridinyl)pyridine, as a yellow solid, m.p. 224—226°C. This product produced a yellow image on contact with acidic clay or phenolic resin.

Example 68

Following a procedure similar to that described in Example 62 but employing 4.7 g. of 2,4-bis(dimethylamino)benzaldehyde, 6.0 g. of 2-acetylthiophene, 32 g. of ammonium acetate and 20 ml. of glacial acetic acid there was obtained 0.6 g. of 4-[2,4-bis(dimethylamino)phenyl]-2,6-bis(2-thienyl)pyridine, as a brown solid, m.p. 73—100°C. This product produced an orange image on contact with acidic clay or phenolic resin.

Example 69

Following a procedure similar to that described in Example 62 but employing 8.8 g. of 2-ethoxy-4-(diethylamino)benzaldehyde, 10.1 g. of 2-acetylthiophene, 52 g. of ammonium acetate and 30 ml. of glacial acetic acid there was obtained 0.6 g. of 4-(2-ethoxy-4-(diethylamino)phenyl]-2,6-bis(2-thienyl)pyridine, as a yellow brown solid, m.p. 91—104°C. This product produced a yellow image on contact with acidic clay or phenolic resin.

Example 70

Following a procedure similar to that described in Example 62 but employing 5.3 g. of the ethyl ester of 1-formyl-5-(dimethylamino)benzoic acid, 5.8 g. of 4-acetylpyridine, 30 g. of ammonium acetate and 25 ml. of glacial acetic acid there was obtained 0.8 g. of 4-[2-(ethoxycarbonyl)-4-(dimethylamino)phenyl]pyridine as a light yellow solid, m.p. 205—215°C. This product produced a yellow image on contact with acidic clay or phenolic resin.

Example 71

Following a procedure similar to that described in Example 62 but employing 2.5 g. of 4-(dimethylamino)benzaldehyde, 6.2 g. of 3-acetyl-2-methylindole, 30 g. of ammonium acetate and 20 ml. of glacial acetic acid there was obtained 0.8 g. of 4-[4-(dimethylamino)phenyl-2,6-bis(2-methylindol-3-yl)pyridine, as a yellow solid, m.p. 171—176°C. This product produced a yellow image on contact with acidic clay or phenolic resin.

Example 72

Following a procedure similar to that described in Example 65 but employing 6.1 g. of 9-julolidinecarboxaldehyde, 7.6 g. of 2-acetylthiophene, 40 g. of ammonium acetate and 30 ml. of glacial acetic acid there was obtained 4.8 g. of 4-(9-julolidinyl)-2,6-bis(2-thienyl)pyridine, as an orange solid, m.p. 198—203°C. This product produced a pinkish yellow image on contact with acidic clay or phenolic resin.

Example 73

A mixture containing 5.6 g. of 4-isoindolinylbenzaldehyde, 6.5 g. of 2-acetylthiophene, 30 g. of ammonium acetate and 40 ml. of glacial acetic acid was refluxed for two hours. After cooling to room temperature, the solid was collected by filtration and washed with water. The solid was then slurried in 150 ml. of ethanol, collected by filtration, and washed successively with 50 ml. portions of ethanol and acetone to give 4.2 g. of 4-(4-isoindolinylphenyl)-2,6-bis-(2-thienyl)pyridine, as a muddy yellow solid, m.p. 185—188°C. This product produced a yellow image on contact with acidic clay or phenolic resin.

Example 74

Following a procedure similar to that described in Example 62 but employing 4.8 g. of 2-methyl-4-(1-pyrrolidinyl)benzaldehyde, 6.5 g. of 2-acetylthiophene, 40 g. of ammonium acetate and 50 ml. of glacial acetic acid there was obtained 1.1 g. of 4-[2-methyl-4-(1-pyrrolidinyl)phenyl]-2,6-bis(2-thienyl)pyridine as a tan solid, m.p. 75—85°C. This product produced an orange image on contact with acidic clay or phenolic resin.

Example 75

Following a procedure similar to that described in Example 62 but employing 9.5 g. of 4-(1-piperidinyl)-benzaldehyde, 13 g. of 2-acetylthiophene, 50 g. of ammonium acetate and 60 ml. of glacial acetic acid there was obtained 3.7 g. of 4-[4-(1-piperidinyl)phenyl]-2,6-bis(2-thienyl)pyridine, as a yellow solid, m.p. 146—149°C. This product produced a yellow image on contact with acidic clay or phenolic resin.

Example 76

Following a procedure similar to that described in Example 65 but employing 6.7 g. of 4-(4-morpholinyl)benzaldehyde, 8.3 g. of 2-acetylthiophene, 50 g. of ammonium acetate and 60 ml. of glacial acetic acid there was obtained 1.8 g. of 4-[4-(4-morpholinyl)phenyl]-2,6-bis(2-thienyl)pyridine, as a yellow solid, m.p. 161—166°C. This product produced a yellow image on contact with acidic clay or phenolic resin.

Example 77

Following a procedure similar to that described in Example 62 but employing 5..6 g. of 1-ethyl-2-methylindole-3-carboxaldehyde, 6.6 g. of 2-acetylfuran, 40 g. of ammonium acetate and 30 ml. of glacial acetic acid there was obtained 1 g. of 4-(1-ethyl-2-methylindol-3-yl)-2,6-bis(2-furyl)pyridine, m.p. 145—150.5°C. This product developed a brownish-yellow image on contact with acidic clay or phenolic resin.

Example 78

Following a procedure similar to that described in Example 62 but employing 7.2 g. of 4-(benzylethylamino)benzaldehyde, 7.3 g. of 4-acetylpyridine, 40 g. of ammonium acetate and 25 ml. of glacial acetic acid there was obtained 3.7 g. of 4-[4-(benzylethylamino)phenyl]-2,6-bis(4-pyridyl)pyridine, m.p. 210—213°C. This product developed a yellow image on contact with acidic clay or phenolic resin.

It is contemplated that by following procedures similar to those described in Examples 62, 63, 65 and 73 but employing the appropriately substituted heteroaryl methyl ketones and aryl or heteroaryl aldehydes of Formulas II and III, respectively, there will be obtained the 4-[4-(substituted-amino)phenyl] or heteroaryl-2,6-bis(heteroaryl)pyridines of Formula I, Examples 79—90 presented in Table A hereinbelow.

TABLE A

| Ex. | Ar | Z |
|---|---|---|
| 79 | 5-$CH_3$-2-furyl | 4-$(CH_3)_2N$—$C_6H_4$ |
| 80 | 5-$CH_3$-2-thienyl | 4-$(CH_3)_2N$—$C_6H_4$ |
| 81 | 2,4-$(CH_3)_2$-2-pyrrolyl | 4-$(CH_3)_2N$—$C_6H_4$ |
| 82 | 2-pyridinyl | 4-$(CH_3)_2N$—$C_6H_4$ |
| 83 | 3-pyridinyl | 4-$(CH_3)_2N$—$C_6H_4$ |
| 84 | 1-$C_2H_5$-2-$CH_3$-3-indolyl | 4-$(C_3H_5)_2N$—$C_6H_4$ |
| 85 | 1-$C_4H_9$-3-indolyl | 4-$(CH_3)_2N$—$C_6H_4$ |
| 86 | 5-$C_4H_9$-2-furyl | 4-$(C_2H_5CH_3N)$—$C_6H_4$ |
| 87 | 5-$C_4H_9$-2-thienyl | 4-$(CH_3)_2N$—$C_6H_4$ |
| 88 | 5-$CH_3O$-1,2-$(CH_3)_2$-3-indolyl | 4-$(CH_3)_2N$—$C_6H_4$ |
| 89 | 2-thienyl | 1-$C_4H_9$-3-indolyl |
| 90 | 2-furyl | 1,2,5-$(CH_3)_3$-3-indolyl |

Example 91

A solution containing 0.73 g. of the color former of Example 75 in 30 g. of isopropylbiphenyl and a solution containing 2.5 g. of carboxymethylcellulose in 100 ml. of water were mixed and emulsified by rapid stirring. The desired particle size (1—4 microns) was checked by microscope. To the emulsion was added a solution containing 7.5 g. of pigskin gelatin in 60 ml. of water. The pH was adjusted to 6.5 with 10% aqueous sodium hydroxide with rapid stirring, and following the gradual addition of 335 ml. of water at 50°C. the pH was adjusted to 4.5 with 10% aqueous acetic acid with continued rapid stirring. After 5 minutes, the mixture was cooled to 100°C., treated with 10 g. of 25% aqueous glutaraldehyde and rapidly stirred for 15 minutes. The resulting microcapsule dispersion was stirred more slowly overnight, diluted with water to 560 g. and coated on white typewriter paper sheets. The sheets were air-dried. Duplicate typewritten images were made on receiving sheets coated with either phenolic resin or acidic clay. The color former of Example 75 produced a yellow image on both types of receiving sheets.

Example 92

A polyvinyl alcohol dispersion of the color former of Example 62 was prepared by shaking one hour on a paint shaker a mixture containing 2.0 g. of the color former, 3.7 g. of water, 8.6 g. of 10% aqueous polyvinyl alcohol and 10 ml. of zirconium grinding beads. A polyvinyl alcohol dispersion of bisphenol A was prepared by shaking a mixture containing 9.8 g. of bisphenol A, 18.2 of water, 42 g. of 10% aqueous polyvinyl alcohol and 70 ml. of zirconium grinding beads. The coating mixture was made by combining and thoroughly mixing 2.1 g. of the polyvinyl alcohol dispersion of the color former with 47.9 g. of the polyvinyl

alcohol dispersion of bisphenol A. The coating mixture was applied to white mimeo paper sheets and the sheets were dried at room temperature. Contacting the coated sheets with a heated stylus at a temperature between 100°C. and 150°C. produced a yellow image.

## Claims

1. A 2,6-bis(heteroaryl)pyridine compound having the formula

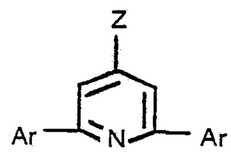

I

wherein:

Ar is a substituent having the formula

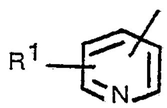

(a)

,

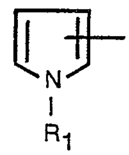

(b)

,

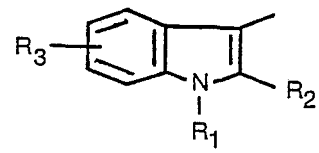

(c)

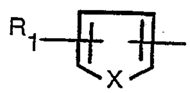

(d)

or

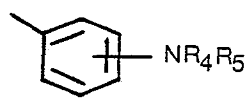

(e)

in which:

$R_1$ is hydrogen or non-tertiary ($C_1$—$C_4$)-alkyl;

$R_2$ is hydrogen, phenyl or non-tertiary ($C_2$—$C_4$-alkyl;

$R_3$ is hydrogen, non-tertiary ($C_1$—$C_4$)-alkyl or non-tertiary ($C_1$—$C_4$)-alkoxy;

$R_4$ and $R_5$ are the same or different and are ($C_1$—$C_4$)-alkyl or benzyl, or $NR_4R_5$ is pyrrolidinyl, piperidinyl, or morpholinyl;

X is O or S;

Z is 9-julolidinyl or a substituent having the formula

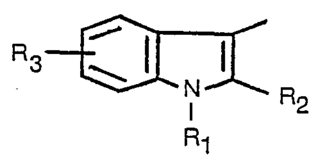

and when Ar is

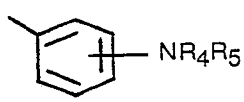

(f)

then Z in addition may be naphthyl or a substituent having the formula

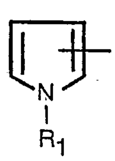

(b)

,

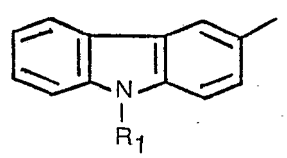

,

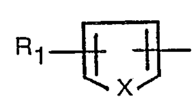

(d)

or

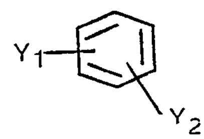

(g)

wherein:

$Y_1$ and $Y_2$ are the same or different and are selected from hydrogen, $(C_1—C_4)$-alkyl, $(C_1—C_4)$-alkoxy, halo, nitro, $(C_1—C_4)$-alkoxy-carbonyl, phenyl or $NR_9R_{10}$;

$R_9$ is $(C_1—C_4)$-alkyl or benzyl;

$R_{10}$ is $(C_1—C_4)$-alkyl, benzyl or cyano-$(C_1—C_4)$-alkyl, or $NR_9R_{10}$ is pyrrolidinyl, piperidinyl, morpholinyl or isoindolinyl; and when Ar is other than

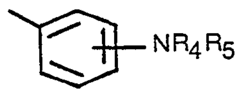

then Z in addition may be

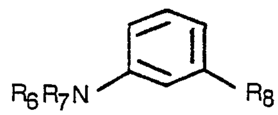

wherein:

$R_6$ is $(C_1—C_4)$-alkyl, and

$R_7$ is $(C_1—C_4)$-alkyl, benzyl, cyano-$(C_1—C_4)$-alkyl or $NR_6R_7$ is pyrrolidinyl, piperidinyl, morpholinyl or isoindolinyl, and

$R_8$ is hydrogen, $(C_1—C_4)$-alkyl, halo, $(C_1—C_4)$-alkoxy, $(C_1—C_4)$-alkoxy-carbonyl or di-$(C_1—C_4)$-alkylamino.

2. A compound according to claim 1, characterized in that Ar is of the formula (f) in which $R_4$ and $R_5$ are each $(C_1—C_4)$-alkyl.

3. A compound according to claims 1 or 2, characterized in that Z is a substituent having the formula (g).

4. A compound according to claim 3, characterized in that at least one of $Y_1$ and $Y_2$ is $NR_9R_{10}$ in which $R_9$ and $R_{10}$ are each $(C_1—C_4)$-alkyl.

5. 2,4,6-Tris[4-(dimethylamino)phenyl]pyridine according to claim 4.

6. A compound according to claim 3, characterized in that $Y_1$ and $Y_2$ are the same or different and are hydrogen, $(C_1—C_4)$-alkyl or $(C_1—C_4)$-alkoxy.

7. 2,6 - bis[4 - Dimethylamino)phenyl] - 4 - phenylpyridine, 2,6 - bis[4 - (dimethylamino)phenyl] - 4 - (4 - methylphenyl)pyridine and 2,6 - bis[4 - (dimethylamino)phenyl] - 4 - (4 - methoxyphenyl) - pyridine according to claim 6.

8. A compound according to claim 1, characterized in that Ar is a substituent of the formula (d) and Z is a substituent having the formula

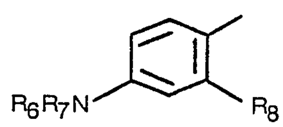

wherein:

$R_6$, $R_7$ and $R_8$ are as defined in claim 1.

9. 4 - [4 - (Dimethylamino)phenyl] - 2,6 - bis(2 - thienyl)pyridine, 4 - [2 - chloro - 4 - (dimethylamino)phenyl] - 2,6 - bis(2 -thienyl)pyridine and 4 - [4 -dimethylamino)phenyl] - 2,6 - bis(2 - furyl)pyridine according to claim 8.

10. A process for producing a compound according to claim 1, characterized by reacting approximately two molar equivalents of a ketone having the formula

$$ArCOCH_3$$

with approximately one molar equivalent of an aldehyde having the formula

$$Z—CHO$$

in the presence of ammonia or an ammonia-releasing agent where in the above formulas Ar and Z are as defined in claim 1.

11. A pressure-sensitive carbonless duplicating system or thermal marking system containing a support sheet coated with a color-forming substance comprising a compound according to any one of claims 1 to 9.

12. A duplicating system or thermal marking system according to claim 11, characterized in that said color-forming substance comprises said compound according to claim 1 in combination with a blue or green and a red or orange color former.

**Revendications**

1. Un composé de type 2,6-bis(hétéroaryl)pyridine répondant à la formule:

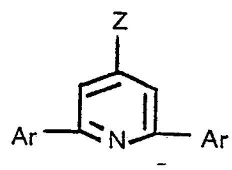

I

dans laquelle

Ar est un substituant de formule:

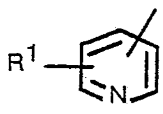

(a)

, 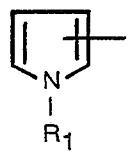

(b)

, 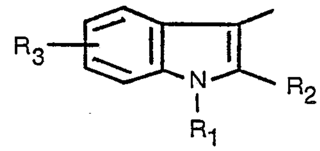

(c)

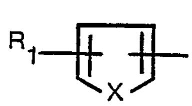

(d)

ou 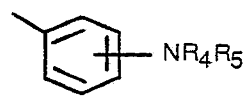

(e)

dans laquelle

$R_1$ est un hydrogène ou un alkyle ($C_1$—$C_4$) non tertiaire;

$R_2$ est un hydrogène, un phényle ou un alkyle ($C_1$—$C_4$) non tertiaire;

$R_3$ est un hydrogène, un alkyle ($C_1$—$C_4$) non tertiaire ou un alcoxy ($C_1$—$C_4$) non tertiaire;

$R_4$ et $R_5$ sont semblables ou différents et sont un alkyle ($C_1$—$C_4$) ou un benzyle, ou $NR_4R_5$ est un pyrrolidinyle, pipéridyle ou morpholinyle;

X est O ou S;

Z est un o-julolidinle ou un substituant de formule:

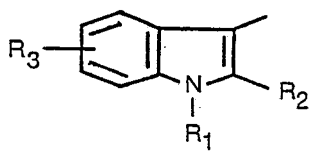

et lorsque Ar est

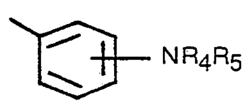

(f)

Z peut en plus être un naphtyle ou un substituant de formule

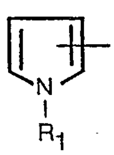

(b)

, 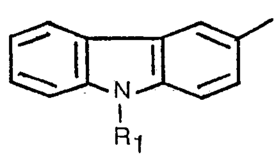

, 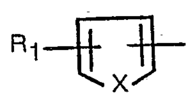

(d)

ou 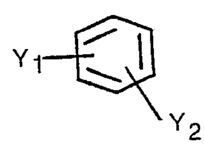

(g)

dans laquelle:

$Y_1$ et $Y_2$ sont semblables ou différents et sont choisis parmi un hydrogène, un alkyle ($C_1$—$C_4$), un alcoxy ($C_1$—$C_4$), un halogéno, un nitro, un alcoxy ($C_1$—$C_4$) carbonyle un phényle ou $NR_9R_{10}$;

$R_9$ est un alkyle ($C_1$—$C_4$) ou un benzyle;

$R_{10}$ est un alkyle ($C_1$—$C_4$), un benzyle ou un cyanoalkyle ($C_1$—$C_4$) ou

$NR_9R_{10}$ est un pyrrolidinyle, un pipéridyle, un morpholinyle ou un isoindolinyle; et lorsque Ar est autre que

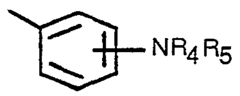

Z peut de plus être

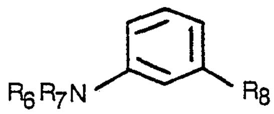

où:

$R_6$ est un alkyle ($C_1$—$C_4$), et

$R_7$ est un alkyle ($C_1$—$C_4$), un benzyle, un cyanoalkyle ($C_1$—$C_4$) ou

$NR_6R_7$ est un pyrrolidinyle, un pipéridyle, un morpholinyle ou un isoindolyle, et

$R_8$ est un hydrogène, un alkyle ($C_1$—$C_4$), un halogéno, un alcoxy ($C_1$—$C_4$), un alcoxy ($C_1$—$C_4$) carbonyle ou un di-alkyl ($C_1$—$C_4$) amino.

2. Un composé selon la revendication 1, caractérisé en ce que Ar répond à la formule (f) dans laquelle $R_4$ et $R_5$ sont chacun un alkyle ($C_1$—$C_4$).

3. Un composé selon les revendications 1 ou 2, caractérisé en ce que Z est un substituant de formule (g).

4. Un composé selon la revendication 3, caractérisé en ce qu'au moins un de $Y_1$ et $Y_2$ est $NR_9R_{10}$ où $R_9$ et $R_{10}$ sont chacun un alkyle ($C_1$—$C_4$).

5. 2,4,6-tris[4-(diméthylamino)phényl]pyridine selon la revendication 4.

6. Un composé selon la revendication 3, caractérisé en ce que $Y_1$ et $Y_2$ sont semblables ou différents et sont un hydrogène, un alkyle ($C_1$—$C_4$) ou un alcoxy ($C_1$—$C_4$).

7. 2,6 - bis[4 - (diméthylamino)phényl] - 4 - phénylpyridine, 2,6 - bis[4 - (diméthylamino)phényl] - 4 - (4 - méthylphényl)pyridine et 2,6 - bis[4 - (diméthylamino)phényl] - 4 - (4 - méthoxyphényl)pyridine selon la revendication 6.

8. Un composé selon la revendication 1, caractérisé en ce que Ar est un substituant de formule (d) et Z est un substituant de formule

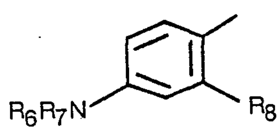

dans laquelle:

$R_6$, $R_7$ et $R_8$ sont comme défini dans la revendication 1.

9. 4 - [4 - (diméthylamino)phényl] - 2,6 - bis(2 - thiényl)pyridine, 4 - [2 - chloro - 4 - (diméthylamino)phényl] - 2,6 - bis(2 - thiényl)pyridine et 4 - [4 - (diméthylamino)phényl] - 2,6 - bis(2 - furyl)pyridine selon la revendication 8.

10. Procédé de préparation d'un composé selon la revendication 1, caractérisé par la réaction d'environ deux équivalents molaires d'une cétone répondant à la formule

$$ArCOCH_3$$

avec environ un équivalent molaire d'un aldéhyde de formule

$$Z—CHO$$

en présence d'ammoniac ou d'un agent libérant de l'ammoniac, où dans les formules ci-dessus Ar et Z sont comme défini dans la revendication 1.

11. Système de duplication sans carbone sensible à la pression ou système de marquage thermique contenant un feuille support revêtue d'une substance chromogène comprenant un composé selon l'une quelconque des revendications 1 à 9.

12. Un système de duplication ou un système de marquage thermique selon la revendication 11 caractérisé en ce que ladite substance chromogène comprend ledit composé selon la revendication 1 en combinaison avec un chromogène bleu ou vert et un chromogène rouge ou orange.

**Patentansprüche**

1. 2,6-Bis(heteroaryl)pyridinverbindung der Formel

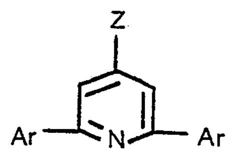

in der:

Ar ein Substituent der Formel

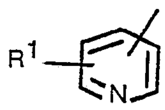 ,

(a)

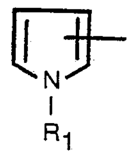 ,

(b)

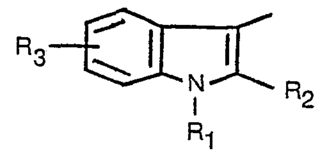

(c)

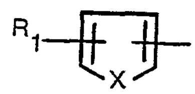 oder 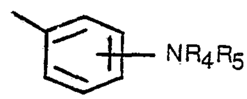

(d) (e)

ist, in der

$R_1$ Wasserstoff oder nichttertiäres ($C_1$—$C_4$)-Alkyl ist;

$R_2$ Wasserstoff, Phenyl oder nichttertiäres ($C_1$—$C_4$)-Alkyl ist;

$R_3$ Wasserstoff, nichttertiäres ($C_1$—$C_4$)-Alkyl oder nichttertiäres ($C_1$—$C_4$)-Alkoxy ist;

$R_4$ und $R_5$ gleich oder verschieden und ($C_1$—$C_4$)-Alkyl oder Benzyl sind, oder $NR_4R_5$ Pyrrolidinyl, Piperidinyl oder Morpholinyl ist;

X die Bedeutung O oder S hat;

Z die Bedeutung 9-Julolidinyl oder eines Substituenten mit der Formel

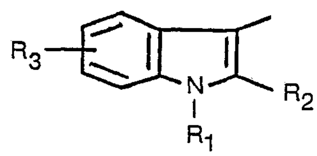

hat und, wenn Ar

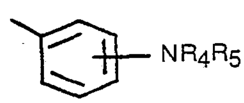 (f)

ist, Z außerdem Naphthyl oder ein Substituent der Formel

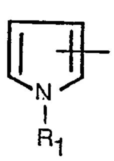 , 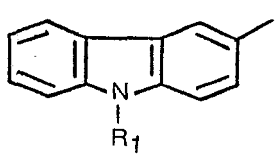 , 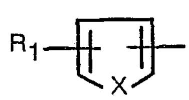 oder 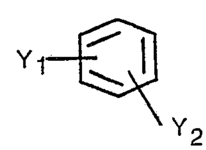

(b) (d) (g)

sein kann, in der

$Y_1$ und $Y_2$ gleich oder verschieden sein können und ausgewählt sind aus Wasserstoff, ($C_1$—$C_4$)-Alkyl, ($C_1$—$C_4$)-Alkoxy, Halogen, Nitro, ($C_1$—$C_4$)-Alkoxy-carbonyl, Phenyl oder $NR_9R_{10}$;

$R_9$ die Bedeutung ($C_1$—$C_4$)-Alkyl oder Benzyl hat;

$R_{10}$ die Bedeutung ($C_1$—$C_4$)-Alkyl, Benzyl oder Cyan-($C_1$—$C_4$)-Alkyl hat oder $NR_9R_{10}$ Pyrrolidinyl, Piperidinyl, Morpholinyl oder Isoindolinyl sit; und wenn Ar etwas anderes ist als

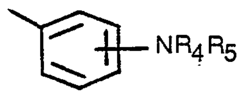 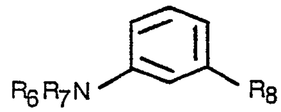

sein kann, wobei

$R_6$ die Bedeutung ($C_1$—$C_4$)-Alkyl und

$R_7$ die Bedeutung ($C_1$—$C_4$)-Alkyl, Benzyl, Cyan-($C_1$—$C_4$)-Alkyl hat oder

$NR_6R_7$ Pyrrolidinyl, Piperidinyl, Morpholinyl oder Isoindolinyl ist und

$R_8$ Wasserstoff, ($C_1$—$C_4$)-Alkyl, Halogen, ($C_1$—$C_4$)-Alkoxy, ($C_1$—$C_4$)-Alkoxy-carbonyl oder DI-($C_1$—$C_4$)-Alkylamino ist.

2. Verbindung nach Anspruch 1, dadurch gekennzeichnet, daß Ar die Formel (f) hat, in der $R_4$ und $R_5$ jeweils ein $C_1$—$C_4$)-Alkyl ist.

3. Verbindung nach Anspruch 1 oder 2, dadurch gekennzeichnet, daß Z ein Substituent mit der Formel (g) ist.

4. Verbindung nach Anspruch 3, dadurch gekennzeichnet, daß mindestens einer der Bestandteile $Y_1$ und $Y_2$ $NR_9R_{10}$ ist, wobei $R_9$ und $R_{10}$ jeweils ein ($C_1$—$C_4$)-Alkyl ist.

5. 2,4,6-Tris[4-(dimethylamino)phenyl]pyridin nach Anspruch 4.

6. Verbindung nach Anspruch 3, dadurch gekennzeichnet, daß $Y_1$ und $Y_2$ gleich oder verschieden sind und Wasserstoff, ($C_1$—$C_4$)-Alkyl oder ($C_1$—$C_4$)-Alkoxy sind.

7. 2,6 - Bis[4 - (dimethylamino)phenyl] - 4 - phenylpyridin, 2,6 - Bis[4 - (dimethylamino)phenyl] - 4 - (4 - methylphenyl) - pyridin und 2,6 - Bis[4 - (dimethylamino)phenyl]4 - (4 - methoxy - -phenyl)pyridin nach Anspruch 6.

8. Verbindung nach Anspruch 1, dadurch gekennzeichnet, daß Ar ein Substituent mit der Formel (d) und Z ein Substituent der mit der Formel

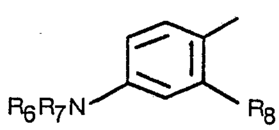

ist, in der

$R_6$, $R_7$ und $R_8$ die im Anspruch 6 angegebene Bedeutungen haben.

9. 4 - [4 - (Dimethylamino)phenyl] - 2,6 - bis(2 - thienyl)pyridin, 4 - [2 - Chlor - 4 - (dimethylamino)-phenyl]2,6 - bis(2 - (thienyl) - pyridin und 4 - [4 - Dimethylamino)phenyl] - 2,6 - bis(2 - furyl)pyridin nach Anspruch 8.

10. Verfahren zum Erzeugen einer Verbindung nach Anspruch 1, dadurch gekennzeichnet, daß etwa zwei Moläquivalente eines Ketons mit der Formel

$$ArCOCH_3$$

mit etwa einem Moläquivalent eines Aldehyds mit der Formel

$$Z—CHO$$

in Gegenwart von Ammoniak oder eines Ammoniak abgebenden Mittels umgesetzt werden, wobei in den angegebenen Formeln Ar und Z die im Anspruch 1 angegebenen Bedeutungen haben.

11. Auf Druck ansprechendes, kohlenstoffreies Vervielfältigungssystem oder thermisches Markiersystem mit einem Trägerblatt, das mit einer farberzeugenden Substanz überzogen ist, die eine Verbindung nach einem der Ansprüche 1 bis 9 enthält.

12. Vervielfältigungssystem oder thermisches Markiersystem nach Anspruch 11, dadurch gekennzeichnet, daß die farberzeugende Substanz die genannte Verbindung nach Anspruch 1 in Kombination mit einer eine blaue oder grüne oder rote orange Farbe erzeugenden Substanz enthält.